# EUROPEAN PATENT APPLICATION

(11) **EP 4 800 118 A1**
(43) Date of publication of application: **02.09.2026**
(21) Application number: 24882324.7
(22) Date of filing: 21.10.2024
(51) Int. Cl.: C12Q 1/02, C07K 14/765, C12N 5/071, G01N 21/64, G01N 33/58, G01N 33/68

(54) **METHOD FOR MEASURING LYSOSOMAL ACTIVITY**

(30) Priority: 24.10.2023 JP 2023182873
(71) Applicant: Oriental Yeast Co., Ltd., Tokyo 174-8505 (JP)
(72) Inventor: IWAI Yoshito, Nagahama-shi, Shiga 526-0804 (JP); FURUYA Yuriko, Nagahama-shi, Shiga 526-0804 (JP); OGURO Yuji, Nagahama-shi, Shiga 526-0804 (JP)
(74) Representative: Hoffmann Eitle
(86) International application number: PCT/JP2024/037305
(87) International publication number: WO 2025/089211

(57) **Abstract**

This invention provides a method that can easily perform measurements of the cellular lysosomal activity in a sample. The method comprises measuring the intracellular lysosomal activity *in vitro* comprising the steps (1) to (4): (1) a step of culturing cells in a cell-comprising medium with the addition of a fluorescent substance-bound protein; (2) a step of collecting the cultured cells after step (1); (3) a step of adding a cell lysis composition to the cultured cells collected in step (2) to obtain a cell lysate fraction; and (4) a step of measuring the fluorescence intensity of the fluorescent substance-bound protein of the cell lysate fraction obtained in step (3).

## Description

### Technical Field

The present invention relates to a method for measuring the lysosomal activity.

### Background Art

A lysosome is an intracellular organelle with a membrane-enclosed acidic area, and it has enzymatic activity of degrading a wide variety of biopolymers with the aid of a protease, glycosidase, lipase, nuclease, or the like. Because it serves as the endpoint of a plurality of pathways, such as endocytosis and autophagy, the lysosome is known to play extensive physiological roles both in direct and indirect manners.

Autophagy is a protein degradation pathway associated with a lysosome. Autophagy is an intracellular bulk degradation system generally observed in eukaryotic cells, which maintains cellular homeostasis through constant replacement of cytoplasmic components. When autophagy is induced in response to a wide variety of stimuli, an autophagosome, which is a double-membrane vesicle, fuses to a lysosome to form an autolysosome, and unwanted proteins are degraded in the autolysosome. Abnormal protein-degrading functions of the autolysosome are known to cause vital phenomena, such as aging and motor dysfunctions, in addition to various diseases, such as cancer, neurodegenerative diseases, cardiovascular diseases, lung diseases, and infectious diseases. In particular, autophagy is reported to play a key role in maintenance of auditory perception and cell configurations.

While various attempts have heretofore been made in order to develop a method for measuring the intracellular lysosomal activity, a uniform method therefor has not yet been established (Patent Literature 1). Examples of classic methods for detecting protease activity of lysosomes include cathepsin maturation assays that are carried out by the pulse-chase method using radioactive isotopes and an immunoblotting method using an anti-cathepsin antibody. While such methods have been extensively employed, some problems are existed in the former method that needs the use of radioactive isotopes, and in the latter method tend to cause errors in assays due to the cell density, the probe concentration, and the uptake rate. In addition, a method involving the use of LysoTracker Dye targeting an acidic content in the lysosome has been known, although such method is not used for quantitative assays (Non Patent Literature 1). As methods for measuring lysosomal activity, a method of quantitative measurement comprising incorporating a fluorescent probe into a plasmid, a method of measurement plasmid-based lysosomal activity using a flow cytometer, and other methods have been reported, although such methods were not easy to perform (Patent Literature 1 and Non Patent Literature 2). Procedures of these techniques are too complicated to attain reliable results, such that different results would be attained every time a measurement is performed, unless a skilled person performs measurements.

As a method for measuring lysosomal activity, a method comprising degrading DQ(Trademark)-BSA comprising bovine serum albumin and a fluorescent dye BODIPY bound thereto in a cell and measuring the emitted fluorescence under microscope observation was developed (Non Patent Literature 3). Since this method requires a measurement under microscope observation, this method was not simple enough to attain highly reliable results with high accuracy, and this method was problematic in terms of, for example, handling of samples, processing of images, acquisition of reliable data on the fluorescence intensity, removal of backgrounds, and improvement in reliability, such as durability over repeated use.

In addition, the conventional techniques described above all presuppose the use of cultured cells for measurement. An analysis using such cultured cells can serve as a convincing tool in selective visualization, such as inspection of localization of a target substance in a cell, on the contrary, such method requires the use of a dedicated-purpose apparatus for microscopic observation, the procedure is complicated, a range of analysis is limited to a defined field, and, accordingly, it is necessary to inspect as to whether or not the entirety is accurately reflected. It is also necessary to examine the viability of the target cells, reliability among batches, the influence of culture media, and other factors.

### Citation List

### Patent Literature

Patent Literature 1: WO 2011/019082

### Non Patent Literature

Non Patent Literature 1: Cytometry Part A, 85A; 169-178, 2014
Non Patent Literature 2: Sci. Rep., 9; 11635, 2019
Non Patent Literature 3: Bio Protoc., 7(19); e2571, 2017

### Summary of Invention

### Object of Invention

The present invention is intended to provide a method that can easily perform measurements of the intracellular lysosomal activity in a sample without performing cell observation using a microscope.

### Means for Achieving the Object

Under above circumstances, the present inventors have conducted concentrated studies, and as a result, found that measurements of lysosomal activity would be performed in a stable and simple manner with high accuracy without being influenced by interfering substances and without performing cell observation by performing cell culture with the addition of a fluorescent substance-bound protein, collecting the cultured cells from a cell culture vessel, lysing the cells with the aid of a cell lysis solution, and measuring the fluorescence intensity.

Specifically, the present invention provides the following.
[1] A method for measuring the intracellular lysosomal activity *in vitro* comprising the steps (1) to (4):
   (1) a step of culturing cells in a medium comprising a fluorescent substance-bound protein;
   (2) a step of collecting the cultured cells after step (1);
   (3) a step of adding a cell lysis composition to the cultured cells collected in step (2) to obtain a cell lysate fraction; and
   (4) a step of measuring the fluorescence intensity of the cell lysate fraction obtained in step (3).
[2] The method according to [1], which further comprises step (5) and step (6) after step (4):
   (5) a step of measuring the protein concentration in the cell lysate fraction; and
   (6) a step of dividing the fluorescence intensity measured in step (4) by the protein concentration measured in step (5) to determine relative fluorescence units (RFU).
[3] The method according to [1] or [2], wherein the fluorescent substance is at least one substance selected from the group consisting of FITC, Cy3, Cy5, and BODIPY.
[4] The method according to any of [1] to [3], wherein the fluorescent substance is BODIPY and the protein is bovine serum albumin (BSA).
[5] The method according to any of [1] to [4], wherein the cell lysis composition is at least one substance selected from the group consisting of CHAPS lysis buffer, HBST, cell lysis buffer, buffer A, M-PER buffer, cell lysis buffer M, and RIPA buffer.
[6] The method according to any of [1] to [5], wherein the cells are at least one of HeLa cells, HEK293 cells, HEI-OC1 cells, and HUVECs.
[7] A method for assisting assessment of the therapeutic efficacy on a target disease comprising measuring the intracellular lysosomal activity in a sample obtained from a subject by the method according to [1].
[8] A kit for measuring the intracellular lysosomal activity *in vitro* comprising:
   a first assay solution comprising a fluorescent substance-bound protein; and
   a second assay solution comprising a cell lysis composition.
[9] The kit according to [8], which further comprises a third assay solution for quantifying the protein concentration.

The description incorporates the contents disclosed by JP Patent Application No. 2023-182873, based on which the priority of the present application claims.

### Advantageous Effects of Invention

The present invention can provide a method that can easily measure the intracellular lysosomal activity in a sample without performing cell observation using a microscope.

### Brief Description of Drawings

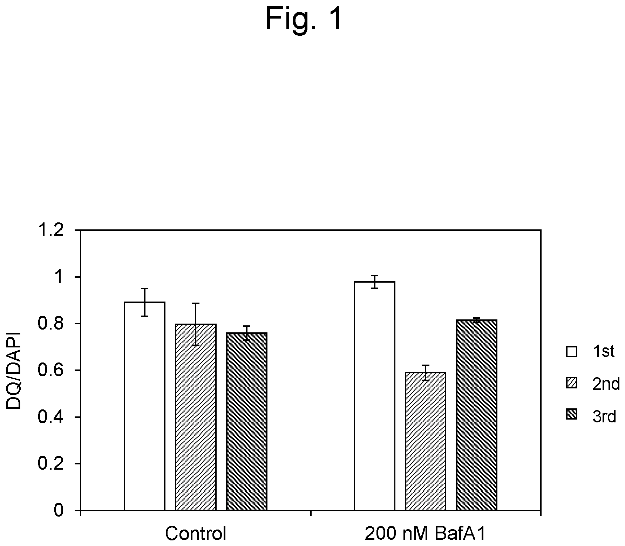

Figure 1 shows a chart demonstrating the results of Comparative Example in which lysosomal activity in the HEK293 cells in the presence of BafA1 was measured by co-staining with DQ Green BSA and DAPI. The tests were performed 3 times on different days, each of the times was conducted with N=4. An error bar in the figure indicates a standard error.

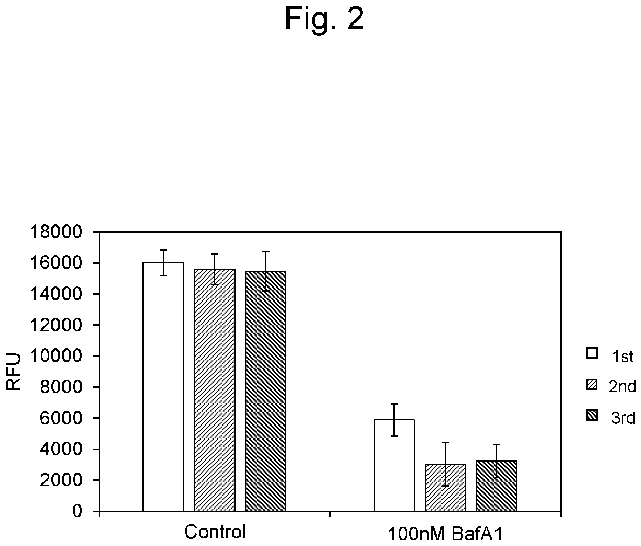

Figure 2 shows a chart demonstrating the results of Example 1 in which lysosomal activity in the HEK293 cells in the presence of BafA1 was measured by staining with DQ Green BSA and performing cell lysis with the use of a cell lysis solution. The tests were performed 3 times on different days, each of the times was conducted with N=3. An error bar in the figure indicates a standard error.

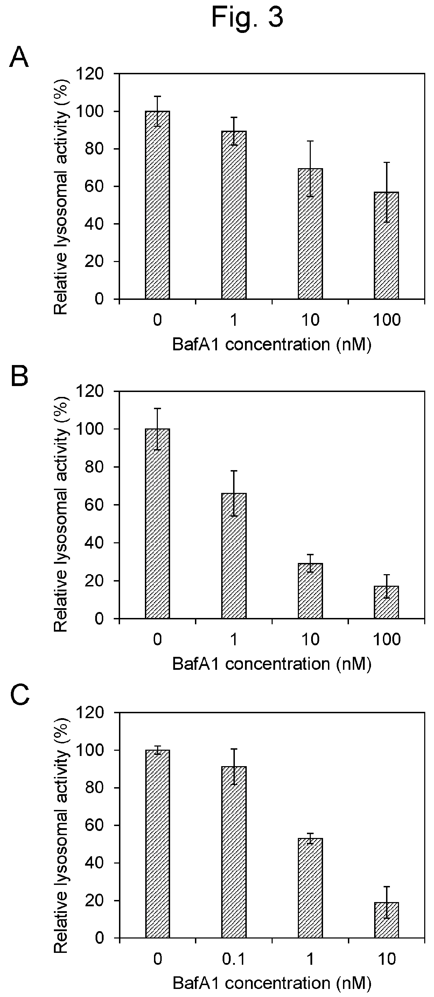

Figure 3 shows a chart demonstrating the results of Example 2 in which lysosomal activity in HeLa cells, HEI-OC1 cells, and HUVECs in the presence of BafA1 at various concentrations was measured by staining with DQ Green BSA and performing cell lysis with the use of a cell lysis solution. A shows lysosomal activity in HeLa cells, B shows lysosomal activity in HE1-OC1, and C shows lysosomal activity in HUVECs. Each of the tests was conducted with N=3. An error bar in the figure indicates a standard error.

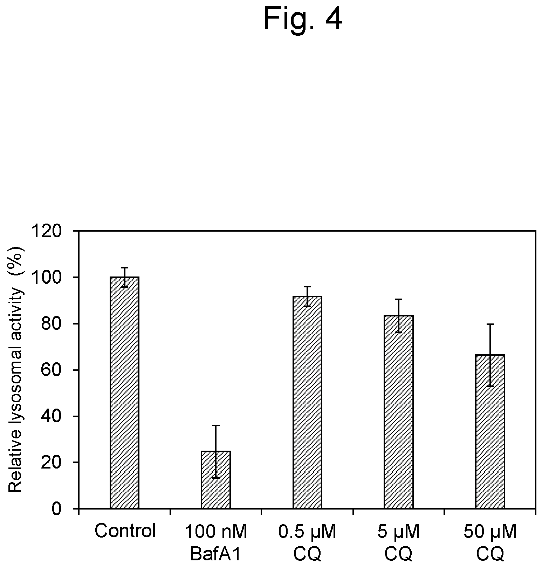

Figure 4 shows a chart demonstrating the results of Example 3 in which lysosomal activity in HEK293 cells in the presence of BafA1 or CQ at various concentrations was measured by staining with DQ Green BSA and performing cell lysis with the use of a cell lysis solution. Each of the tests was conducted with N=3. An error bar in the figure indicates a standard error.

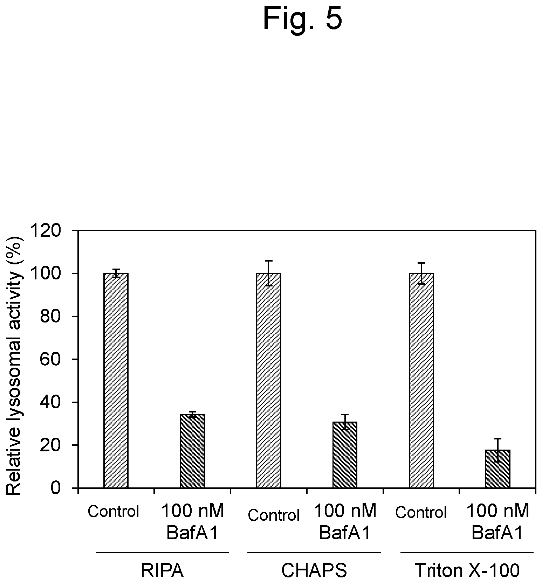

Figure 5 shows a chart demonstrating the results of Example 4 in which lysosomal activity in HEK293 cells was measured by performing cell lysis with the use of CHAPS buffer and Triton X-100 buffer. Each of the tests was conducted with N=3. An error bar in the figure indicates a standard error.

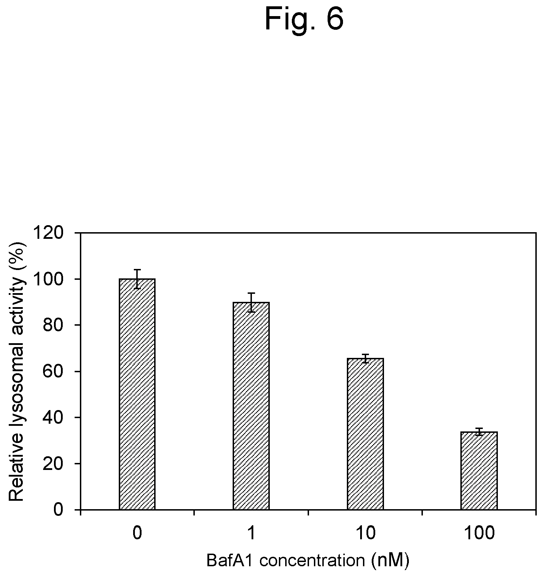

Figure 6 shows a chart demonstrating the results of Example 5 in which lysosomal activity in HEK293 cells in the presence of BafA1 was measured by staining with DQ ovalbumin and performing cell lysis with the use of a cell lysis solution. Each of the tests was conducted with N=3. An error bar in the figure indicates a standard error.

### Description of Embodiments

### 1. Components and definition

### <Sample>

A "sample" is not particularly limited herein, provided that the sample comprises cells, which can serve as targets of lysosomal activity measurements, and examples thereof include all organs, tissue, and cells. Specific examples of samples include organs, tissue, and cells derived from vertebrates, such as mammals, birds, reptiles, amphibians, and fishes. A sample is preferably derived from a mammal and it is more preferably derived from a human or experimental animal (such as a mouse, rat, hamster, or rabbit). The "cells" are not particularly limited herein, provided that the cells comprise proteins, and the cells may be in any forms or may be derived from any specimens, such as cell lines, primary cultured cells, or tissue-derived sections. In particular, cultured cells are preferably used. The "tissue" is not particularly limited herein, and tissue that is cultured under special conditions, such as cancer tissue or ischemic tissue, may be used herein. The sample may be obtained from tissue or cells or the environment of tissue or cells. In some examples, a sample can be a tissue biopsy material, blood, plasma, extracellular fluid, cultured cell, medium, disposed tissue, plant-derived material, synthetic protein, archaebacteria, bacteria, fungal tissue, or protozoan. Examples of cultured cells and primary cultured cells include, but are not limited to, the human cervical carcinoma cells (HeLa cells), the human embryonic kidney cells (HEK293 cells), mouse cochlear hair cells (HEI-OC1 cells), and human umbilical vein endothelial cells (HUVECs).

### <Fluorescent substance-bound protein>

The term "fluorescent substance-bound protein" used herein refers to a protein comprising a fluorescent substance, which is also referred to as a "self-quenching dye," bound thereto, and such protein is constituted to emit fluorescence upon incorporation thereof into a lysosome and hydrolysis of its protein portion. A protein constituting a "fluorescent substance-bound protein" is not particularly limited, and, for example, bovine serum albumin (BSA) is preferably used. The term "fluorescent substance" used herein refers to a substance, such as an organic compound or protein that emits fluorescence of a particular fluorescent wavelength upon application of a light of a particular excitation wavelength. Examples of compounds known as organic compounds include FITC (Molecular Probes), Texas Red (Molecular Probes), Cy3 and Cy5 (GE HealthCare Technologies Inc.), borondipyrromethene dyes (BODIPY dyes), such as 4,4-difluoro-4-bora-3a,4a-diaza-s-indacene (BODIPY^{(™)}) and BDP-FL, BIP, CF-MONO, CF-BI, and BDPFL NHS-ester. Examples of known proteins include GFP, CFP, RFP and YFP. Use of BODIPY FL and BODIPY TR is preferable.

As a "fluorescent substance-bound protein," for example, a commercially available product can be used. An example of such commercially available product is DQ-BSA comprising BSA and BODIPY bound thereto (DQ-BSA, Thermo Fisher).

### <Cell lysis composition>

As a "cell lysis composition," a solution that is generally used for cell lysis or protein solubilization can be used herein. For example, a solution that is known as CHAPS lysis buffer, RIPA buffer, HBST, Cell lysis buffer, Tissue lysis buffer, or Buffer A can be used. In general, such solution is also referred to as a "cell lysis reagent," "cell lysis liquid," or the like. A person skilled in the art can select and use an adequate composition. The cell lysis composition may comprise a buffer. These buffers having buffering action at around weakly alkaline levels, such as PBS, Tris, or HEPES, can be used.

The cell lysis composition may comprise a surfactant, such as CHAPS, SDS, NP40, or Triton-X, for protein solubilization. It may comprise urea having solubilization effect, and an adequate material may be selected and used.

The cell lysis composition may comprise a protease inhibitor so as to avoid protein degradation caused by proteases in cells. The protease inhibitor may be adequately selected from among generally used substances, such as phenylmethanesulfonyl fluoride (PMSF), aprotinin, leupeptin, pepstatin, sodium fluoride, and sodium orthovanadate, and used. A divalent metal ion may be used as a sequestering agent (a chelating agent) in order to inhibit proteases. Examples of chelating agents that can be used include EDTA and EGTA. In order to maintain a protein in a phosphorylated state, a phosphatase inhibitor, such as sodium fluoride, sodium orthovanadate, sodium pyrophosphate, or β-glycerophosphoric acid, may be used.

Protein disulfide bonds may be cleaved with the use of a reducing agent, such as DTT (dithiothreitol) or BME (β-mercaptoethanol).

A person skilled in the art can determine adequate concentrations of these additives comprised in the cell lysis composition and prepare to use these additives. In the case of RIPA buffer, for example, the cell lysis composition may comprise 50 mM Tris-HCl (pH 7.4), 150 mM NaCl, 1% Triton X-100 or NP-40, 0.5% sodium deoxycholate, 0.1% SDS, 1 mM EDTA, and 10 mM NaF. In accordance with applications, the composition may be adequately modified, or an additive, such as a protease inhibitor, may be added. A person skilled in the art can adequately modify the composition and prepare to use the composition in accordance with the purpose of the experiment. As a commercially available product, for example, M-PER buffer (Thermo Fisher) or cell lysis buffer M (FUJIFILM Wako Pure Chemical Corporation) may be used. Use of RIPA buffer is preferable.

The term "kit" used herein refers to an assembly of a plurality of elements used to implement a method. The term "reagent" used herein refers to a substance of a single element. A reagent comprised in a vessel consists of a single element.

### 2. Method for measuring lysosomal activity in cell

The first embodiment of the present invention relates to a method for measuring the intracellular lysosomal activity. The method of the embodiment comprises measuring the intracellular lysosomal activity *in vitro* comprising the steps (1) to (4):
(1) a step of culturing cells in a medium comprising a fluorescent substance-bound protein;
(2) a step of collecting the cultured cells after step (1);
(3) a step of adding a cell lysis composition to the cultured cells collected in step (2) to obtain a cell lysate fraction; and
(4) a step of measuring the fluorescence intensity of the fluorescent substance-bound protein of the cell lysate fraction obtained in step (3).

The method of the embodiment comprises a step of lysing cells when measuring the intracellular lysosomal activity and this enables quantitative measurement of enzymatic activity of the lysosome without a complicated procedure, such as image analysis. Thus, the lysosomal activity can easily be measured with high accuracy regardless of the skill of a person implementing the measurements.

### <Step (1) Step of culture>

Hereafter, each step of the method of the embodiment is described. Hereafter, the invention is described with reference to a step of measuring the lysosomal activity with the use of the HEK293 cells, although the scope of the invention is not limited thereto.

Step (1) of the method of the embodiment comprises adding a fluorescent substance-bound protein to a medium comprising cells and culturing the cells. Cells are preferably subjected to pre-culture in a cell culture dish or the like prior to step (1). Pre-culture is performed, for example, with the use of the DMEM medium at 37°C for 24 hours or longer.

The medium used for pre-culture is replaced with a medium comprising a fluorescent substance-bound protein and cell culture is continued. The concentration of the fluorescent substance-bound protein is not particularly limited, it can be 1 to 100 µg/ml or 5 to 50 µg/ml, and, in particular, it can be approximately 10 µg/ml. Culturing temperature is preferably 30°C to 40°C, and it is particularly preferably 32°C to 38°C. A culturing period is preferably 10 minutes to 24 hours, and it is particularly preferably 30 minutes to 8 hours. The culturing temperature and the culturing period can be adequately adjusted in accordance with the type or properties of the cells used.

### <Step (2) Step of collection>

Step (2) of the method of the embodiment comprises collecting the cultured cells after step (1). The cells may be collected with the use of a protease, such as trypsin, depending on a cell type. For example, cultured cells can be collected by performing centrifugation at 4°C and 1000× g (approximately 2,000 rpm) for 5 minutes. The cells may be washed with ice-cooled PBS, and the number of the cells may be counted.

### <Step (3) Step of cell lysis>

Step (3) of the method of the embodiment comprises adding a cell lysis composition to the cultured cells collected in step (2) to obtain a cell lysate fraction. A step involving the use of RIPA buffer as the cell lysis composition is exemplified herein.

To the cells collected in step (2), ice-cooled RIPA buffer is added. In this case, the RIPA buffer preferably comprises a protease inhibitor. It is preferable that 100 µl of the RIPA buffer be added to the collected 10⁶ cells and when the protein concentration is deduced to be higher because of the cell type or conditions, for example, the volume of the RIPA buffer may be adequately increased or decreased. A person skilled in the art can adequately determine the amount of the RIPA buffer to be used. Thereafter, the RIPA buffer comprising the cells may be stored on the ice for 30 minutes while repeating vortex mixing, and the resultant may be used for measurement of lysosomal activity.

When cells as the measured targets are derived from tissue, the target tissue is subjected to anatomy, and when it is necessary to remove blood, the tissue is washed with cool PBS and used. After the tissue is cut into pieces while cooling on the ice, the tissue is introduced into a homogenizer, and the RIPA buffer comprising a protease inhibitor is added. The amount of the RIPA buffer used may be, for example, 500 µl relative to approximately 10 mg of the tissue. The RIPA buffer comprising tissue may be completely homogenized using a homogenizer, allowed to stand on the ice for 30 minutes, and then used for measurement of lysosomal activity. According to need, a step of ultrasonic disruption (sonication) may be performed. In the step of sonication, the sample is ultrasonically treated, and the cells or tissue is further disrupted to cleave DNA. A period of ultrasonic treatment can be adequately modified in accordance with a cell type. While ice-cooling the sample, for example, at 180 watts, cell lysis may be performed for approximately 1 minute, and tissue lysis may be performed for approximately 2 to 5 minutes (a cycle of 10 seconds of ultrasonication and 10 seconds of quiescence is repeated).

### <Step (4) Step of measuring fluorescence>

Step (4) of the method of the present embodiment comprises measuring the fluorescence intensity of the fluorescent substance-bound protein of the cell lysate fraction obtained in step (3). A method for measuring the fluorescence intensity of the cell lysate fraction is not particularly limited, and a conventional means, such as a plate reader or flow cytometer, can be adequately used. In order to easily perform measurements within a short period, it is preferable that the fluorescence intensity be measured with the use of a plate reader. Hereafter, a step involving the use of a plate reader is exemplified.

The cell lysate fraction extracted with the use of the cell lysis composition is added to a black 96-well plate for luminescent/fluorescent analysis, and the fluorescence intensity is measured using a plate reader (e.g., BMG Labtech) (at the excitation wavelength of 485 nm and the fluorescent wavelength of 520 nm when DQ-BSA is used), and by this enables measurement of lysosomal activity. Each sample may be subjected to a single instance of measurement or a plurality of instances of measurements. In order to perform measurement of lysosomal activity with higher accuracy, measurement is preferably performed multiple times. The measured fluorescence intensity may be corrected with the use of, for example, the protein concentration, so that the lysosomal activity can be measured with higher accuracy. For example, the fluorescence intensity of the RIPA buffer as the background may be subtracted from the fluorescence intensity and the determined value may be employed.

### <Step (5) Step of measuring protein concentration>

The method of the embodiment may further comprise step (5) and step (6) after step (4). Step (5) of the method of the embodiment comprises measuring the protein concentration in the cell lysate fraction. A method for measuring the protein concentration is not particularly limited, and any known method can be employed, for example, a general method based on the BCA method can be employed. According to the BCA method, quantification can be performed with the use of the Pierce BCA Protein Assay Kit (Thermo Fisher Scientific).

### <Step (6) Step of determining RFU>

Step (6) of the method of the present embodiment comprises dividing the fluorescence intensity measured in step (4) by the protein concentration measured in step (5) to determine relative fluorescence units (RFU). By indicating the lysosomal activity in terms of RFU, changes in the lysosomal activity can be assessed with higher accuracy when, for example, assessing the influence of a drug on the lysosome.

### <Application Example>

The method of the embodiment comprises the components and the steps described above, andsuch components and steps enable measurement of intracellular lysosomal activity in a simple manner, and thus, the method of the embodiment is expected to contribute to an achievement in research on various diseases including cancers and neurodegenerative diseases, aging, and motor dysfunctions that are considered to be associated with lysosomal storage disorders and autophagy. In addition, the method of the embodiment enables effective use of the invention for assessment of dependency of the disease on drug concentration, screening of therapeutic agents, verification of the validity of therapeutic measures of various diseases as the indicator, and other purposes. In particular, it is possible to employ the method of the embodiment to observe the therapeutic effects to assess the therapeutic efficacy on a disease.

An application example of the method of the embodiment is a method for screening for a drug that enables recovery from lysosomal disorders. The present inventors found that the intracellular lysosomal activity measured in the method of the embodiment would be lowered in a manner dependent on the lysosome inhibitor concentration in the presence of cells and a lysosome inhibitor. Such discovery demonstrates that the method of the embodiment enables quantitative assessment of an extent of lysosomal disorders and an extent of recovery therefrom. Such screening method can comprise, for example, steps (a) to (d) below:
(a) a step of culturing cells in a medium comprising a candidate drug, a lysosome inhibitor, and a fluorescent substance-bound protein;
(b) a step of collecting the cultured cells after step (a);
(c) a step of adding a cell lysis composition to the cultured cells collected in step (b) to obtain a cell lysate fraction; and
(d) a step of measuring the fluorescence intensity of the fluorescent substance-bound protein of the cell lysate fraction obtained in step (c).

An extent of the recovery of the lysosomal activity lowered by the action of the lysosome inhibitor in step (a) achieved with the aid of the candidate drug is examined in steps (b) to (d), and thus, a potential of the candidate drug to recover the lysosomal activity can be examined in a simple manner.

As a "lysosome inhibitor," for example, known lysosome inhibitors, such as bafilomycin A1 (hereafter, may be referred to as "BafA1") and chloroquine (hereafter, may be referred to as "CQ"), can be used. In addition, any substances having activity of damaging a lysosome, such as hydroxychloroquine having a structure similar to that of chloroquine, bisbenzylisoquinoline alkaloid, such as azithromycin, and macrolide antibiotics, such as tetrandrine, can be used.

### 3. Method for assisting assessment of therapeutic efficacy on disease

The second embodiment of the invention relates to a method for assisting assessment of the therapeutic efficacy on a target disease. The method of the embodiment comprises measuring the intracellular lysosomal activity in a sample obtained from a subject by the method of the first embodiment. The reagent, the apparatus, the samples, the conditions, and the like employed in the method of the embodiment are the same as those employed in the first embodiment, unless it is otherwise described or unless there is inconsistency.

The intracellular lysosomal activity can be used to assess the therapeutic efficacy as the indicator to verify the validity of the therapeutic measures of various diseases. Because numerous enzymes and proteins related thereto exist in a lysosome, when dysfunction such as lowering in activity of such enzymes occurs, accordingly, lysosomal diseases, such as sphingolipidosis, mucopolysaccharidosis, oligosaccharidosis, glycogenosis, neutral lipid accumulation, and I-cell disorder (mucolipidosis II), are known to be induced. There are 40 or more types of such inborn errors of metabolism that induce a wide variety of symptoms, and these diseases are "designated intractable diseases" in Japan. The method of the embodiment can be used to obtain the indicator to verify the validity of the therapeutic measures of diseases, such as the lysosomal disease, that are known to be highly associated with the cellular lysosomal activity. For example, a sample obtained from a subject is subjected to measurement of lysosomal activity *in vitro*, and the measured value is compared with the control value, and thus, the measurement results can be used as the indicator to determine as to whether the dose of the therapeutic agent should be increased or decreased.

For example, a given dose of a therapeutic agent is administered to a target patient who has been afflicted with a disease known to be associated with the lysosomal activity, the intracellular lysosomal activity of the patient is measured, and the measured value is compared with the preset cut-off value or the control value before administration of the therapeutic agent, andthus, whether the dose of the therapeutic agent should be increased or decreased can be determined. When the lysosomal activity is lower than the preset cut-off value or the control value before administration of the therapeutic agent, for example, the dose of the therapeutic agent may be changed to be higher than the original dose of the therapeutic agent and applied to the target patient. When the lysosomal activity is higher than the preset cut-off value or the control value before administration of the therapeutic agent, for example, the dose of the therapeutic agent may be changed to be lower than the original dose of the therapeutic agent and applied to the target patient.

### 4. Kit for measurement of lysosomal activity

The third embodiment of the invention relates to a kit for measuring the intracellular lysosomal activity *in vitro.* The kit of the embodiment comprises a first assay solution comprising a fluorescent substance-bound protein and a second assay solution comprising a cell lysis composition. More specifically, the kit of the embodiment is used in the method of the first embodiment. The reagent, the apparatus, the samples, the conditions, and the like employed for the kit of the embodiment are the same as those employed in the first embodiment, unless it is otherwise described or unless there is inconsistency.

The kit of the embodiment comprises at least two elements; i.e., a first assay solution comprising a fluorescent substance-bound protein and a second assay solution comprising a cell lysis composition. The first assay solution can be a medium comprising the fluorescent substance-bound protein. Alternatively, the first assay solution may be a buffer comprising the fluorescent substance-bound protein at high concentration. The second assay solution may be a cell lysis composition (e.g., RIPA buffer) or a 5× concentrated solution of the cell lysis composition (to be diluted before use).

The kit of the embodiment may comprise a third assay solution, which is a composition for quantifying the protein concentration. The composition for quantifying the protein concentration may be any known composition for quantifying the protein concentration. When protein quantification is performed by the BCA method, for example, the kit may comprise a bicinchoninic acid solution and a copper sulfate solution.

The kit of the embodiment may further comprise a cell culture dish, a black microplate for measurement of fluorescence, an instruction for use (a package insert), and the like, according to need.

### Examples

### [Comparative Example] Measurement of intracellular lysosomal activity by co-staining with DQ Green BSA and DAPI

HEK293 cells were diluted in the DMEM medium and seeded at 2 × 10⁴ cells/well on 8-well chamber slides. The seeded cells were cultured at 37°C in the presence of 5% CO₂ for 24 hours, the culture supernatant was removed, and 0 (the control), 200 nM bafilomycin A1 (BafA1, Bioaustralis), and the DMEM medium comprising 10 µg/ml DQ Green BSA were added, followed by incubation at 37°C for 6 hours. The supernatant was removed, and the cells were fixed with 1% paraformaldehyde and stained with DAPI. The image was obtained using a fluorescent microscope (Keyence) (the exposure time: 4 seconds for GFP; 1/200 seconds for DAPI), the threshold was standardized to 19-255 in Image J, and a green fluorescence was calculated as an area. The calculated value was divided by the number of DAPI-stained cell nuclei, and a chart was prepared using the obtained value (Figure 1). Groups each consisting of N = 4 were subjected to sampling. The tests were performed 3 times on different days. An error bar in the figure indicates a standard error.

Figure 1 shows the measurement results of the 3 tests. Variation in the measured values was large depending on the days of testing, and it was accordingly impossible to clearly detect lowering in the lysosomal activity caused by the addition of BafA1. This method of measurement is considered to be insufficient in terms of accuracy and reproducibility because the visual fields in wells are selected manually when obtaining images using a fluorescent microscope and variations among persons implementing tests or variations among samples would become large.

### [Example 1] Measurement of intracellular lysosomal activity using DQ Green BSA and cell lysis solution

HEK293 cells were diluted in the DMEM medium and seeded at 3 × 10⁵ cells/well on a 6-well plate. The seeded cells were cultured at 37°C in the presence of 5% CO₂ for 24 hours, the culture supernatant was removed, and 0 (the control), 100 nM BafA1, and the DMEM medium comprising 10 µg/ml DQ Green BSA were added, followed by incubation at 37°C for 6 hours. The supernatant was removed, the cells were collected, and RIPA buffer (50 mM Tris-HCl, 0.1% SDS, 0.5% DOC, 1% NP-40, 150 mM NaCl (pH 8.0)) was added, followed by ice-cooling for 10 minutes. Thus, proteins were extracted from the cells to obtain an extract.

The extract was fractionated at 150 µl/well to a black 96-well plate for luminescent/fluorescent analysis, and the fluorescence intensity (the excitation wavelength: 485 nm; the fluorescent wavelength: 520 nm) was measured using a plate reader (BMG Labtech). Also, proteins comprised in the extract were quantified using the Pierce BCA Protein Assay Kit (Thermo Fisher Scientific).

The fluorescence intensity of the RIPA buffer as the background was subtracted from the fluorescence intensity of the sample with relevant BafA1 concentration, and the obtained value was divided by the protein concentration in the extract (RFU). Groups each consisting of N = 3 were subjected to sampling. The tests were performed 3 times on different days.

Figure 2 shows the measurement results of the 3 tests. An error bar in the figure indicates a standard error. The results demonstrate that an error depending on the days of testing is small and the measurement results with reliability higher than that of Comparative Example would be attained. The results also demonstrate that it is possible to clearly detect lowering in the lysosomal activity caused by BafA1 . Such achievement is considered to be realized because signal measurements can be performed collectively without manually selecting the visual field unlike the measurement using a fluorescent microscope.

### [Example 2] Dependency of measured value of lysosomal activity on BafA1 concentration

With the use of HeLa cells, HEI-OC1 cells, and HUVECs, dependency of measured value of lysosomal activity on the BafA1 concentration was examined. HeLa cells, HEI-OC1 cells, and HUVECs were diluted in the DMEM medium and seeded at 3 × 10⁵ cells/well on a 6-well plate. The seeded HeLa cells and HUVECs were cultured at 37°C in the presence of 5% CO₂ for 24 hours, the seeded HEI-OC1 cells were cultured at 33°C in the presence of 10% CO₂ for 24 hours, the culture supernatant was removed, 0, 1, 10, or 100 nM BafA1 and the DMEM medium comprising 10 µg/ml DQ Green BSA were added to HeLa cells and to HEI-OC1 cells, 0, 0.1, 1, or 10 nM BafA1 and the DMEM medium comprising 10 µg/ml DQ Green BSA were added to HUVECs, HeLa cells and HUVECs were incubated at 37°C for 6 hours, and HEI-OC1 cells were incubated at 33°C for 1 hour. Thereafter, preparation of extracts, and measurements of fluorescence, background, and protein concentration were performed in the same manner as in Example 1. The background was subtracted from the measured value of fluorescence of each sample, and the obtained value was divided by the protein concentration to calculate RFU. The RFU value of the sample not comprising BafA1 was designated to be 100%, the relative lysosomal activity (%) was calculated based on the RFU value of the sample comprising BafA1, and the chart was prepared based thereon (Figure 3). Groups each consisting of N = 3 were subjected to sampling.

Figure 3 shows dependency of the lysosomal activity on BafA1 concentration of each cell. Figure 3 A shows the results of HeLa cells, Figure 3 B shows the results of HEI-OC1 cells, and Figure 3 C shows the results of HUVECs. An error bar in the figure indicates a standard error. As shown in the figure, it was demonstrated that the lysosomal activity would be lowered in a manner dependent on the BafA1 concentration in all of HeLa cells, HEI-OC1 cells, and HUVECs. The results also demonstrate that the method of the present invention enables detection of damage on the lysosomal activity in a drug concentration-dependent manner and different drug-susceptibility depending on cells.

### [Example 3] Dependency of lysosomal activity on concentration of other inhibitors

By the method of the present invention, dependency of the intracellular lysosomal activity on the BafA1 concentration and on the chloroquine (CQ) concentration was examined in the same manner as in Example 2. HEK293 cells were diluted in the DMEM medium and seeded at 3 × 10⁵ cells/well on a 6-well plate. The seeded cells were cultured at 37°C in the presence of 5% CO₂ for 24 hours, the culture supernatant was removed, and 100 nM BafA1 or 0.5, 5, or 50 µM CQ and the DMEM medium comprising 10 µg/ml DQ Green BSA were added, followed by incubation at 37°C for 6 hours. As the control, incubation was performed in the same manner with the addition of a medium not comprising the inhibitor. Thereafter, preparation of extracts, and measurements of fluorescence, background, and protein concentration were performed in the same manner as in Example 1. The background was subtracted from the measured value of fluorescence of each sample, and the obtained value was divided by the protein concentration to calculate RFU. The control RFU value was designated to be 100%, the relative lysosomal activity (%) was calculated based on the RFU value of the sample comprising BafA1 or CQ, and the chart was prepared based thereon (Figure 4). Groups each consisting of N = 3 were subjected to sampling.

Figure 4 shows the results. An error bar in the figure indicates a standard error. It was demonstrated that the lysosomal activity would be lowered in a concentration-dependent manner not only in the presence of BafA1 but also in the presence of CQ. Both BafA1 and CQ are drugs that are reported to damage the lysosome. That is, the measured value of lysosomal activity obtained by the method of the present invention reflects the actual lysosomal activity.

### [Example 4] Measurement of lysosomal activity using various cell lysis solutions

HEK293 cells were subjected to measurements of lysosomal activity using various cell lysis solutions. HEK293 cells were diluted in the DMEM medium and seeded at 3 × 10⁵ cells/well on a 6-well plate. The seeded cells were cultured at 37°C in the presence of 5% CO₂ for 24 hours, the culture supernatant was removed, and 0 (the control), 100 nM BafA1, and the DMEM medium comprising 10 µg/ml DQ Green BSA were added, followed by incubation at 37°C in the presence of 5% CO₂ for 6 hours. The supernatant was removed, the cells were collected, and RIPA buffer (50 mM Tris-HCl, 0.1% SDS, 0.5% DOC, 1% NP-40, 150 mM NaCl (pH 8.0)), CHAPS buffer (50 mM Tris-HCl, 150 mM NaCl, 1% CHAPS (pH 7.6)), and Triton X-100 buffer (50 mM Tris-HCl, 150 mM NaCl, 50 mM EDTA, 1% Triton X-100 (pH 7.6)) were added, followed by ice-cooling for 10 minutes. Thereafter, collection of extracts, and measurements of fluorescence, background, and protein concentration were performed in the same manner as in Example 1. The background was subtracted from the measured value of fluorescence of each sample, and the obtained value was divided by the protein concentration to calculate RFU. The RFU value of the sample not comprising BafA1 was designated to be 100%, the relative lysosomal activity (%) was calculated based on the RFU value of the sample comprising BafA1, and the chart was prepared based thereon (Figure 5). Groups each consisting of N = 3 were subjected to sampling.

Figure 5 shows the lysosomal activity measured with the use of various cell lysis solutions in the presence of BafA1 and in the absence of BafA1. It was demonstrated that the lysosomal activity was lowered by BafA1 regardless of the type of the buffer used.

### [Example 5] Measurement of intracellular lysosomal activity using DQ ovalbumin and cell lysis solution

HEK293 cells were subjected to measurement of lysosomal activity using DQ ovalbumin and a cell lysis solution. HEK293 cells were diluted in the DMEM medium and seeded at 3 × 10⁵ cells/well on a 6-well plate. The seeded cells were cultured at 37°C in the presence of 5% CO₂ for 24 hours, the culture supernatant was removed, and 0, 1, 10, or 100 nM BafA1 and the DMEM medium comprising 10 µg/ml DQ ovalbumin were added, followed by incubation at 37°C in the presence of 5% CO₂ for 6 hours. Thereafter, cell lysis, collection of extracts, and measurements of fluorescence, background, and protein concentration were performed in the same manner as in Example 1. The background was subtracted from the measured value of fluorescence of each sample, and the obtained value was divided by the protein concentration to calculate RFU. The RFU value of the sample not comprising BafA1 was designated to be 100%, the relative lysosomal activity (%) was calculated based on the RFU value of the sample comprising BafA1, and the chart was prepared based thereon (Figure 6). Groups each consisting of N = 3 were subjected to sampling.

Figure 6 shows the lysosomal activity measured with the use of DQ ovalbumin. An error bar in the figure indicates a standard error. It was demonstrated that the lysosomal activity would be lowered in a manner dependent on the BafA1 concentration with the use of DQ ovalbumin as with the use of DQ Green BSA.

### Industrial Applicability

The present invention is applicable in the medical, pharmaceutical production, and other fields.

All publications, patents, and patent applications cited herein are incorporated herein by reference in their entirety.

## Claims

1. A method for measuring the intracellular lysosomal activity *in vitro* comprising the steps (1) to (4):
(1) a step of culturing cells in a medium comprising a fluorescent substance-bound protein;
(2) a step of collecting the cultured cells after step (1);
(3) a step of adding a cell lysis composition to the cultured cells collected in step (2) to obtain a cell lysate fraction; and
(4) a step of measuring the fluorescence intensity of the cell lysate fraction obtained in step (3).

2. The method according to claim 1, which further comprises step (5) and step (6) after step (4):
(5) a step of measuring the protein concentration in the cell lysate fraction; and
(6) a step of dividing the fluorescence intensity measured in step (4) by the protein concentration measured in step (5) to determine relative fluorescence units (RFU).

3. The method according to claim 1, wherein the fluorescent substance is at least one substance selected from the group consisting of FITC, Cy3, Cy5, and BODIPY.

4. The method according to claim 1, wherein the fluorescent substance is BODIPY and the protein is bovine serum albumin (BSA).

5. The method according to claim 1, wherein the cell lysis composition is at least one substance selected from the group consisting of CHAPS lysis buffer, HBST, Cell lysis buffer, Buffer A, M-PER buffer, Cell lysis buffer M, and RIPA buffer.

6. The method according to claim 1, wherein the cells are at least one of HeLa cells, HEK293 cells, HEI-OC1 cells, and HUVECs.

7. A method for assisting assessment of the therapeutic efficacy on a target disease comprising measuring the intracellular lysosomal activity in a sample obtained from a subject by the method according to claim 1.

8. A kit for measuring the intracellular lysosomal activity *in vitro* comprising:
a first assay solution comprising a fluorescent substance-bound protein; and
a second assay solution comprising a cell lysis composition.

9. The kit according to claim 8, which further comprises a third assay solution for quantifying the protein concentration.
